(19) Europäisches Patentamt
European Patent Office
Office européen des brevets

(11) Publication number: **0 084 417**
**B1**

(12) **EUROPEAN PATENT SPECIFICATION**

(45) Date of publication of patent specification: **07.05.86**

(21) Application number: **83300093.8**

(22) Date of filing: **07.01.83**

(51) Int. Cl.[4]: **C 07 C 45/53,** C 07 C 49/82, C 07 C 49/794, C 07 C 45/66, C 07 C 47/52

(54) **Production of aromatic carbonyl compounds including novel acetophenones.**

(30) Priority: **07.01.82 JP 1573/82**
**12.08.82 JP 140658/82**
**28.10.82 JP 190567/82**

(43) Date of publication of application:
**27.07.83 Bulletin 83/30**

(45) Publication of the grant of the patent:
**07.05.86 Bulletin 86/19**

(84) Designated Contracting States:
**DE FR GB IT NL**

(56) References cited:
**DE-A-2 010 534**
**DE-A-2 631 324**
**FR-A-1 049 001**
**FR-A-2 231 652**
**GB-A- 641 250**
**US-A-2 557 968**

**The file contains technical information submitted after the application was filed and not included in this specification**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Harada, Haruhisa**
**No. 23-5, Aobadai 7-chome**
**Ichihara-shi Chiba (JP)**
Inventor: **Maki, Hiroshi**
**No. 1353-4, Shiizu**
**Ichihara-shi Chiba (JP)**

(74) Representative: **Moore, Anthony John et al**
**Gee & Co. Chancery House Chancery Lane**
**London WC2A 1QU (GB)**

(56) References cited:
**US-A-2 634 294**
**US-A-2 671 111**
**US-A-3 401 193**

**TETRAHEDRON, vol. 35, no. 17, 1979, pages 2021-2026, Pergamon Press, G.B. Z. CEKOVIC et al.: "Remote functionalisation by ferrous ion-cupric ion induced decomposition of alkyl hydroperoxides"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 084 417 B1

Courier Press, Leamington Spa, England.

**Description**

This invention relates to a method of producing aromatic carbonyl compounds comprising decomposing the corresponding hydroperoxide.

The well known conventional methods of synthesizing carbonyl compounds by the decomposition of hydroperoxide are as follows:

(1) Decomposition by heating in the presence of an aqueous alkali solution.

(2) Decomposition with potassium ferrocyanide [*J.A.C.S., 75*,4268 (1953)].

(3) Decomposition with potassium ferrous salt ($FE^{++}$) [J. Org. *Chem., 15* 763 (1950), etc].

(4) Decomposition of organic hydroperoxide with a metal reducing agent to obtain alcohols and ketones [U.S. Patent 2,557,968].

(5) Decomposition of tetralin hydroperoxide with a water-soluble inorganic metal salt to obtain α-tetralone [German Patent 2,631,324].

With method (1), however, large quantities of alcohol compounds are produced, so that carbonyl compounds cannot be obtained in high yields.

Although, according to the cited reference, method (2) produces acetophenone from cumene hydroperoxide in very high yield, because this reaction is almost stoichiometric, a large quantity of expensive potassium ferrocyanide needs to be used. Consequently, this method is not always industrially advantageous.

Method (3) is described widely in the literature and according, for example, to the description in the reference cited above, acetophenone is obtained in a yield of 71% by the decomposition of cumene hydroperoxide with a ferrous salt ($Fe^{++}$). In this method, however, the amount of the salt used is generally large, and the yield is low, so it is again disadvantageous from an industrial point of view.

In method (4), there is a description that α,α-dimethylbenzyl hydroperoxide is decomposed with a ferrous salt to obtain acetophenone. According to this method, the reaction is carried out approximately at room temperature using singly the respective metal salt, but in case it is desired to obtain acetophenone in a high yield, the amount of the metal salt used is as high as several mols per the hydroperoxide. Further, when the amount of catalyst used is equimolar or less, the reaction rate is markedly slow and the yield of acetophenone is very low. Accordingly, when such method is employed, there are serious drawbacks in that the cost of catalyst is expensive from the industrial viewpoint, and that the sludge formation is considerable, resulting in an increased cost for the waste liquor treatment.

In method (5), a specific concentration of terralin hydroperoxide is decomposed with a water-soluble inorganic metal salt (inorganic iron salts or inorganic copper salts or mixtures thereof, according to the disclosure) under a specified condition to obtain α- tetralone with a high selectivity. But according to this method, side reactions are likely to take place. Accordingly, although this method is a decomposition method of tetralin hydroperoxide, when the condition for decomposition is applied to other aromatic hydroperoxides, there are problems in that the reaction rate is slow, the selectivity of the resulting aromatic carbonyl compound is not satisfactory, and that formation of catalyst precipitate occurs so that a filtration step is required in order to remove the precipitate. Thus this method is quite disadvantageous from the industrial viewpoint.

We have found that aromatic carbonyl compounds can be obtained in markedly high yields and with a rapid de-composition rate by decomposing a hydroperoxide under an inert gas atmosphere and in the presence of an aqueous layer containing iron sulfate, copper sulfate and sulfuric acid.

According to the present invention there is provided a method of producing an aromatic carbonyl compound represented by one of the following general formulae (B)-1 and (B)-2:

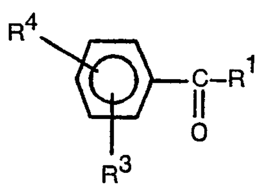

(B)–1

and

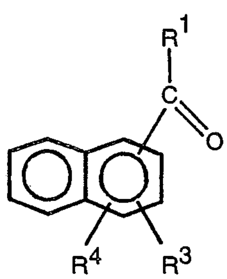

(B)–2

wherein $R^1$ is a hydrogen atom or a methyl group; and $R^3$ and $R^4$ are each a hydrogen atom, a ($C_1$-$C_3$) alkyl group,

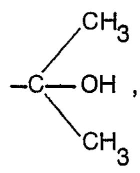 , 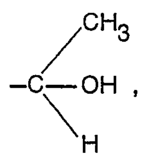 , 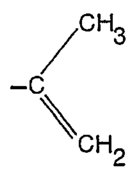 , 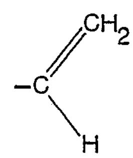 , $-COCH_3$ , 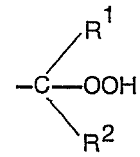 ,

-COOH or a carboxylic acid ester group, comprising decomposing a corresponding hydroperoxide represented by the following general formulae (A)-1 or (A)-2:

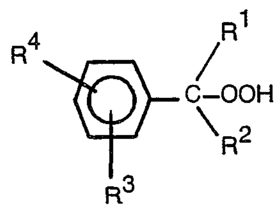

(A)-1

and

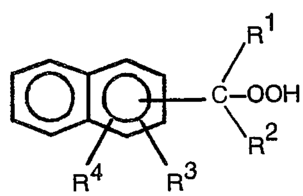

(A)-2

wherein $R^2$ is a hydrogen atom or a methyl group; and $R^1$, $R^3$ and $R^4$ are as previously defined, under an inert gas atmosphere in the presence of an aqueous layer containing iron sulfate — copper sulfate and sulfuric acid.

Specific examples of the hydroperoxide starting materials represented by the formula (A)-1 and (A)-2 include cumene hydroperoxide, cymene hydroperoxide, diisopropylbenzene dihydroperoxide, ethylcumene hydroperoxide, diisoproplylbenzene monohydroperoxide, diethylbenzene monopropylbenzene monohydroperoxide, diethylbenzene monohydroperoxide, *m*-(2-hydroxy-2-propyl) cumene hydroperoxide, isopropenylcumene hydroperoxide, ethylbenzene hydroperoxide, (α-hydroxyethyl)ethylbenzene hydroperoxide, acetylcumene hydroperoxide, triisopropylbenzene trihydroperoxide, methyl cuminate hydroperoxide and isopropylnaphthalene hydroperoxide.

Specific examples of the product aromatic carbonyl compound represented by the formulae (B)-1 and (B)-2 include acetophenone, methylacetophenone, ethylacetophenone, isopopylacetophenone, *m*-(2-hydroxy-2-propyl)-acetophenone, *m*-isopropenylacetophenone, (α-hydroxyethyl)-acetophenone, vinylacetophenone, diacetylbenzene, acetylcumene hydroperoxide, triacetylbenzene, methyl acetylbenzoate and acetylnaphthalene. Of these compounds, *M*-(2-hydroxy-2-propyl)acetophenone and *m*-isopropenylacetophenone are useful as intermediates for the preparation of medicines, such as, for example, phenylephrine hydrochloride represented by the formula:

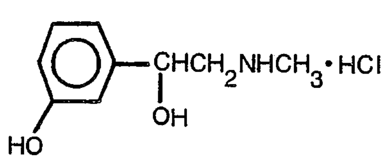

which is known as a vasoconstrictor.

The production of the hydroperoxides represented by the formulae (A)-1 and (A)-2 is not particularly limited, but a method generally used in industry is air-oxidation of a corresponding compound represented by the following general formula (C)-1 or (C)-2:

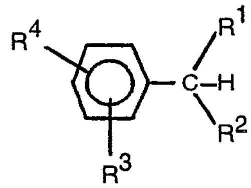

(C)-1

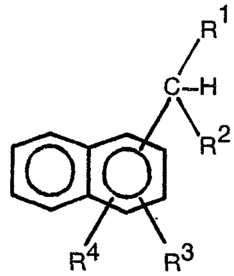

(C)-2

wherein $R^1$, $R^2$, $R^3$ and $R^4$ are as previously defined above. Thus, the corresponding hydroperoxide represented by the formulae (A)-1 or (A)-2 can be easily obtained.

The hydroperoxide can be used alone or in admixture with one or more other defined hydroperoxides.

The method of the present invention is carried out under an inert gas atmosphere such as nitrogen or helium: under an aerial or oxygen atmosphere, the metal sulfate is oxidized, the reaction rate slows down, sludge formation increases and the yield of aromatic carbonyl compound is lowered; consequently such an atmosphere is undesirable.

In the method of the present invention, the co-presence of both iron sulfate and copper sulfate in the aqueous catalytic solution is essential. The use of iron sulfate alone has defects such as that the yield of the aromatic carbonyl compound is low; and that, although the decomposition of the hydroperoxide is initially very fast, in order to obtain a good overall decomposition of the hydroperoxide, the reaction has to be prolonged for a long period of time or the amount of the iron sulfate used has to be increased.

The amount of the iron sulfate which may be used is from 0.005 to 0.5 mole per mole of the hydroperoxide group of the hydroperoxide, and the amount of the copper sulfate which may be used is from 0.05 to 3 moles per mole of the iron sulfate.

When the amount of the iron sulfate is less than 0.005 mole per mole of the hydroperoxide group, the reaction rate is slow, and because of side reactions, the formation of alcohols is enhanced; such a small amount is therefore disadvantageous. On the other hand, when the amount is more than 0.5 mole, the reaction rate is increased, but because of side reactions, the formation of phenols or heavy substances is enhanced; such a large amount is therefore again dis-advantageous. When the amount of copper sulfate used is less than 0.05 mole per mole of the iron sulfate, there is no effect from its co-presence and, hence, no improvement in the yield of aromatic carbonyl compound is observable. When the amount is more than 3 moles, a degree of improvement in the yield is observable, but the cost of catalyst is increased undesirably.

Further features of the method of the present invention are that ferrous sulfate and/or ferric sulfate may be used as the iron sulfate; and that cuprous sulfate and/or cupric sulfate may be used as the copper sulfate.

Sulfuric acid has the effect of inhibiting the formation of sludge.

In the method of the present invention, the hydroperoxide may be used as it is, but, in order to cause the reaction to proceed smoothly, it is more preferred to employ the hydroperoxide in solution in an appropriate organic solvent layer containing the hydroperoxide. Examples of suitable organic solvents which may be used include benzene, toluene, xylene, methyl isobutyl ketone and compounds represented by the formula (C)-1 or (C)-2.

The ratio of hydroperoxide-containing organic layer to the aqueous layer containing the iron salt, copper salt and acid is adjusted so that the amount of the aqueous layer is not less than 10 parts, preferably not less than 20 parts, per 100 parts by weight of the organic layer.

When the proportion of the aqueous layer is less than 10 parts, the reaction rate is decreased, and because of side reactions, the formation of phenols or heavy substances is enhanced.

Furthermore, in the method of the present invention, the aqueous layer containing the iron salt, copper salt and acid can be re-used after completion of the reaction by oily layer-aqueous layer separation. This is not possible in the prior art method employing only ferrous salt.

The reaction temperature is generally selected from the range of 30° to 100°C, preferably 40° to 90°C. When the reaction temperature is less than 30°C, the reaction rate is slow, and when it exceeds 100°C, side reactions increase, both such effects being undesirable.

The reaction is generally carried out under atmospheric pressure, but it may also be carried out under reduced pressure. Furthermore, the reaction may be carried out batchwise or continuously.

After completion of the reaction, the compound represented by the formula (B)-1 or (B)-2 precipitates as a solid from the reaction mixture, or is recovered as an oily layer after the oily layer-aqueous layer separation. When a higher purity is required, it can be obtained by purification by the usual methods such as recrystallization or distillation. When organic solvent is used for the reaction, the compound can be recovered by separating the oily layer from the aqueous layer, removing the organic solvent by one of the usual methods such as distillation, and then distilling the bottom fraction under reduced pressure.

The present invention will now be illustrated in detail by the following Examples, which are not, however, to be interpreted as limiting the invention.

Example 1

Into a 5-litre separable flask was charged 1,000 g of a methyl isobutyl ketone solution containing 19.9wt% of *m*-(2-hydroxy-2-propyl) cumene hydroperoxide (containing 1.32 moles of hydroperoxide group), and the temperature was raised to 80°C while passing a nitrogen gas stream. After the temperature of the contents of the flask had reached 80°C, 1,000 g of an aqueous solution containing 18.3 g of a ferrous sulfate hydrate (containing 0.066 mole of $Fe^{++}$), 10.5 g of copper sulfate (containing 0.066 mole of $Cu^{++}$) and 3.3 g of concentrated sulfuric acid was added thereto from a dropping funnel, and the reaction was carried out at 80°C. After 3 hours' reaction, it was found that the concentration of the remaining hydroperoxide was not more than 0.1 wt%, and hence, the reaction was substantially complete and sludge formation was not substantially observed. Gas chromatographic analysis showed that the yield of *m*-(2-hydroxy-2-propyl) acetophenone was 92%.

Comparative examples 1 to 3

Comparative experiments were carried out in the same manner as in Example 1 except that the reaction conditions were varied as shown in Table 1. The results are shown in Table 1.

TABLE 1

| | Reaction | | | Condition | | | | |
|---|---|---|---|---|---|---|---|---|
| | Atmosphere | Temperature (°C) | Time (hr) | Iron Salt (g(mol)) | Copper Salt (g(mol)) | Acid (g(mol)) | Yield (%) | Remarks |
| Example 1 | $N_2$ | 80 | 3 | Ferrous Sulfate<br>18.3 (0.066) | Copper Sulfate<br>10.5 (0.066) | Sulfuric Acid<br>3.3 (0.033) | 92 | Sludge formation was not substantially observed. |

Examples 2 to 6

Using a methyl isobutyl ketone solution of *m*-(2-hydroxy-2-propyl) cumene hydroperoxide, the reaction was carried out in the same manner as in Example 1 except that the amount of ferrous sulfate per mole of a hydroperoxide group and that of copper sulfate per mole of ferrous sulfate were varied as shown in Table 2. The results are shown in Table 2.

TABLE 2

| Example No. | (1) $Fe^{++}$/Hydroperoxide Group | (2) $Cu^{++}/Fe^{++}$ | (3) Reaction Time (hr) | (4) Yield (%) |
|---|---|---|---|---|
| 2 | 0.05 | 0.1 | 6 | 88 |
| 3 | 0.05 | 0.5 | 4 | 90 |
| 4 | 0.05 | 2.0 | 3 | 93 |
| 5 | 0.1 | 0.1 | 3 | 88 |
| 6 | 0.1 | 1.0 | 1.5 | 91 |

Note: All reactions were carried out while passing a nitrogen gas stream.
(1) The number of moles of ferrous sulfate per mole of hydroperoxide group.
(2) The number of moles of copper sulfate per mole of ferrous sulfate.
(3) A period of time which passed until the concentration of the remaining hydroperoxide reached 0.1 wt% or less.
(4) Yield of *m*-(2-hydroxy-2-propyl) acetophenone.

Comparative Example 1

Into a 5-litre separable flask was charged 1,000 g of a methyl isobutyl ketone containing 19.9 wt% of *m*-(2-hydroxy-2-propyl) cumene hydroperoxide and 1,000 g of an aqueous layer containing 93 g of a ferric sulfate hydrate (containing 0.3 mole of $Fe^{+++}$), and the temperature was raised to 80°C while passing a nitrogen gas stream. The reaction was carried out at 80°C for 9 hours, but it was found that a considerable amount of the hydroperoxide remained undecomposed and hence no substantial reaction took place.

Example 7

The reaction was carried out in the same manner as in Example 1 except that 18.5 g of a ferric sulfate hydrate (containing 0.066 mole of $Fe^{+++}$) was used in place of the ferrous sulfate hydrate. When ferric sulfate was used, the decomposition rate of the hydroperoxide was somewhat slower than with the ferrous sulfate, but the concentration of the remaining hydroperoxide was not more than 0.1 wt% after 4.5 hours' reaction. The yield of *m*-(2-hydroxy-2-propyl) acetophenone was 87%.

Examples 8 to 14

The reaction was carried out in the same manner as in Example 1 except that the various hydroperoxides as shown in Table 3 were used in place of *m*-(2-hydroxy-2-propyl) cumene hydroperoxide. The reaction conditions and the results obtained are also shown in Table 3.

TABLE 3

| Example No. | Hydroperoxide | Reaction Conditions: Temperature (°C) | Time (hr) | Ferrous Sulfate (g) | Copper Sulfate (g) | Yield (%) | Product |
|---|---|---|---|---|---|---|---|
| 8 | Cumene hydroperoxide | 80 | 3 | 19.2 | 11.0 | 92 | Acetophenone |
| 9 | Cymene hydroperoxide | 80 | 3 | 18.4 | 10.6 | 90 | Methylacetophenone |
| 10 | Ethylbenzene hydroperoxide | 80 | 2 | 21.1 | 12.1 | 94 | Acetophenone |
| 11 | *m*-Diisopropylbenzene dihydroperoxide | 80 | 3.5 | 24.6 | 14.1 | 85 | *m*-Diacetylbenzene |
| 12 | 1,3,5-Triisopropylbenzene trihydroperoxide | 80 | 4 | 27.8 | 15.9 | 78 | 1,3,5-Triacetylbenzene |
| 13 | Methyl cuminate hydroperoxide | 80 | 3 | 13.2 | 7.6 | 91 | Methyl acetylbenzoate |
| 14 | ß-Isopropylnaphthalene hydroperoxide | 80 | 3 | 13.7 | 7.9 | 90 | ß-Acetylnaphthalene |

Note:
(1) The hydroperoxides were used as a 20 wt% solution in the solution in the solvents described below, and the amount of the solution used was 1,000 g: cumene for Example 9; cymene for Example 10; ethylbenzene for Example 11; methyl isobutyl ketone for Example 12; triisopropylbenzene for Example 13; and methyl isobutyl ketone for Example 14 and 15.
(2) The aqueous layer was an aqueous solution containing ferrous sulfate, copper sulfate and concentrated sulfuric acid of 0.5 mole per mole of ferrous sulfate, and its amount was 1,000 g.

Example 15

Recyling of the aqueous layer recovered in Example 1 was carried out as follows:-

(1) The procedure of Example 1 was carried out;

(2) 1,000 g of fresh methyl isobutyl ketone solution containing *m*-(2-hydroxy-2-propyl) cumene hydroperoxide (starting material) was used for every recycled use;

(3) to the recovered aqueous layer were added, for every recycled use, fresh ferrous sulfate hydrate of one-fifth of the amount in Example 1, fresh sulfuric acid of 0,5 mole per mole of fresh ferrous sulfate and a fresh copper sulfate of one-tenth of the same; and

(4) the reaction was run 3 hours so that the concentration of the remaining hydroperoxide was not more than 0.1 wt%. The results of five successive recyclings showed that the yield of *m*-(2-hydroxy-2-propyl) acetophenone was not less than 90% for each recycled use and hence that recycling of the aqueous layer was possible.

**Claims**

1. A method of producing an aromatic carbonyl compound represented by one of the following general formulae (B)-1 and (B)-2

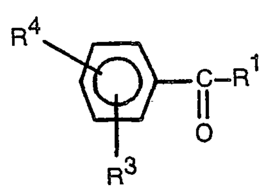

(B)-1

and

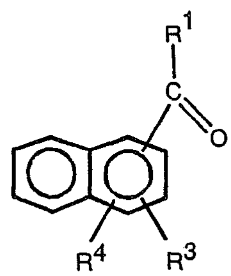

(B)-2

wherein $R^1$ is a hydrogen atom or a methyl group; and $R^3$ and $R^4$ are each a hydrogen atom, a $(C_1$-$C_3)$ alkyl group,

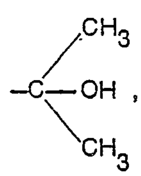 , 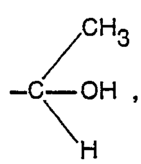 , 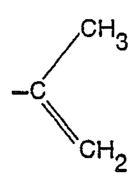 , 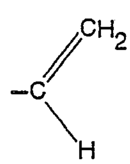 , $-COCH_3$ , 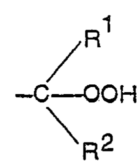 ,

-COOH or a carboxylic acid ester group, comprising decomposing a corresponding hydroperoxide represented by the following general formulae (A)-1 or (A)-2:

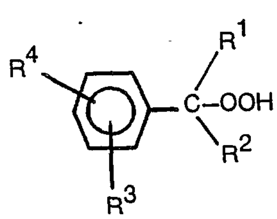

(A)-1

and

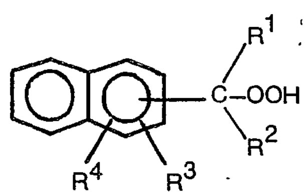

(A)-2

wherein $R^2$ is a hydrogen atom or a methyl group; and $R^1$, $R^3$ and $R^4$ are as previously defined, under an inert gas atmosphere in the presence of an aqueous layer containing iron sulfate, copper sulfate and sulfuric acid.

2. A method as claimed in Claim 1, wherein the iron sulfate is present in an amount of from 0.005 to 0.5 mole per mole of the hydroperoxide group of said hydroperoxide, and the copper sulfate is present in an amount of from 0.05 to 3 moles per mole of the iron salt.

3. A method as claimed in Claim 1 or 2, wherein the hydroperoxide is used as a solution in an organic solvent.

4. A method as claimed in Claim 3, wherein the weight of the aqueous layer containing the iron sulfate, copper sulfate and sulfuric acid is not less than 10 parts per 100 parts by weight of the organic solvent layer containing the hydroperoxide.

**Patentansprüche**

1. Verfahren zur Herstellung einer aromatischen Carbonylverbindung der folgenden allgemeinen Formel (B)-1 oder (B)-2

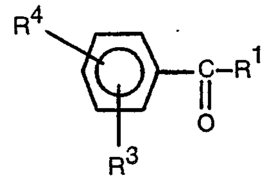

(B)–1

und

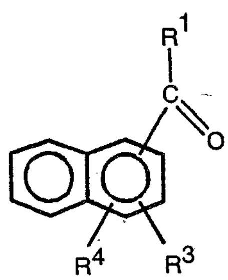

(B)–2

in denen $R^1$ ein Wasserstoffatom oder eine Methylgruppe bedeutet und $R^3$ und $R^4$ jeweils ein Wasserstoffatom, einen $C_1$-$C_3$-Alkyl-rest,

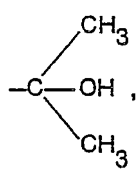 , 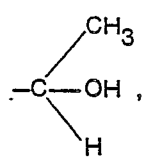 , 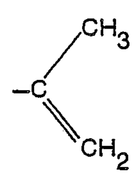 , 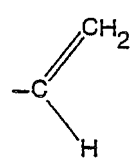 , $-COCH_3$ , 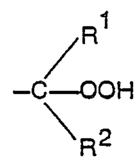 ,

eine -COOH-Gruppe oder einen Carbonsäureesterrest bedeuten, dadurch gekennzeichnet, daß man ein entsprechendes Hydroperoxid der allgemeinen Formel (A)-1 oder (A)-2:

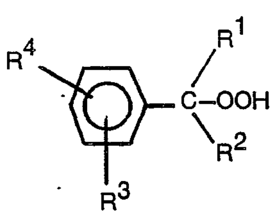

(A)–1

und

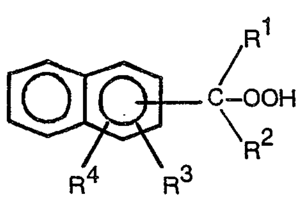

(A)–2

in denen $R^2$ ein Wasserstoffatom oder eine Methylgruppe bedeutet und $R^1$, $R^3$ und $R^4$ die vorstehend angegebene Bedeutung haben, in einer Inertgasatmosphäre in Gegenwart einer wäßrigen Schicht, die Eisensulfat, Kupfersulfat und Schwefelsäure enthält, zersetzt.

2. Verfahren nach Anspruch 1, bei dem das Eisensulfat in einer Menge von 0,005 bis 0,5 Mole pro Mol der Hydroperoxid-gruppe des vorgenannten Hydroperoxids und das Kupfersulfat in einer Menge von 0,05 bis 3 Mole pro Mol des Eisensalzes vorliegt.

3. Verfahren nach den Ansprüchen 1 oder 2, bei dem das Hydroperoxid als Lösung in einem organischen Lösungsmittel verwendet wird.

4. Verfahren nach Anspruch 3, bei dem das Gewicht der das Eisensulfat, Kupfersulfat und Schwefelsäure enthaltenden wäßrigen Schicht nicht weniger als 10 Teile pro 100 Gewichtsteile der das Hydroperoxid enthaltenden organischen Lösungsmittelschicht ausmacht.

**Revendications**

1. Procédé de production d'un composé carbonylé aromatique représenté par l'une des formules générales (B)-1 et (B)-2 suivantes:

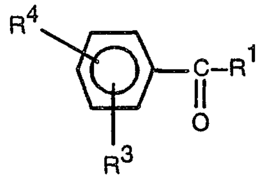

(B)-1

et

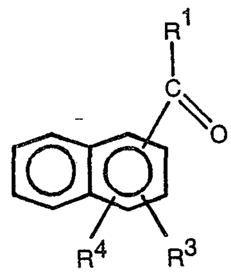

.

(B)-2

dans lesquelles $R^1$ est un atome d'hydrogène ou un groupe méthyle et $R^3$ et $R^4$ sont chacun un atome d'hydrogène, un groupe alkyle en $C_1$-$C_3$,

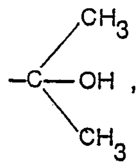 , 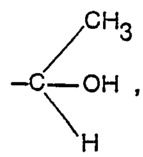 , 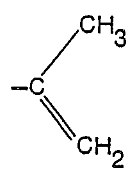 , 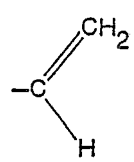 , $-COCH_3$ , 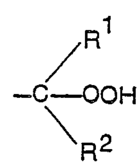 ,

-COOH ou un groupe ester d'acide carboxylique, comprenant la décomposition d'un hydroperoxyde correspondant représenté par la formule générale (A)-1 ou (A)-2 suivante dans lesquelles:

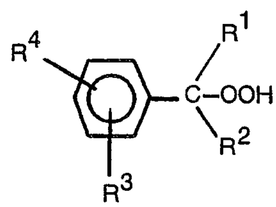

(A)-1

et

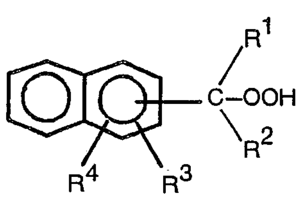

(A)-2

dans lesquelles $R^2$ est un atome d'hydrogène ou un groupe méthyle et $R^1$, $R^3$ et $R^4$ sont comme définis précédemment, en atmosphère de gaz inerte en présence d'une couche aqueuse contenant du sulfate de fer, du sulfate de cuivre et de l'acide sulfurique.

2. Procédé selon la revendication 1, dans lequel le sulfate de fer est présent en quantité de 0.005 à 0.5 mole par mole du groupe hydroperoxyde dudit hydroperoxyde et le sulfate de cuivre est présent en quantité de 0.05 à 3 moles par mole du sel de fer.

3. Procédé selon la revendication 1 ou 2, dans lequel l'hydroperoxyde est utilisé en solution dans un solvant organique.

4. Procédé selon la revendication 3, dans lequel le poids de la couche aqueuse contenant le sulfate de fer, le sulfate de cuivre et l'acide sulfurique est de pas moins de 10 parties par 100 parties en poids de la couche de solvant organique contenant l'hydroperoxyde.